# FASCICULE DE BREVET EUROPEEN

(11) **EP 3 944 763 B1**
(45) Date de publication et mention de la délivrance du brevet: **17.07.2024**
(21) Numéro de dépôt: 21186872.4
(22) Date de dépôt: 21.07.2021
(51) Int. Cl.: A01N 25/34, A01N 63/50, A01N 37/46, C07C 15/20, C07K 19/00, C09D 189/00, A01P 1/00

(54) **ENSEMBLE D ATOMES DE CARBONE A L ETAT D HYBRIDATION SP2 FONCTIONNALISÉ, SON PROCEDE DE PREPARATION ET SES UTILISATIONS, NOTAMMENT POUR RENDRE UNE SURFACE ANTIBACTERIENNE**
KOHLENSTOFFATOM-ANORDNUNG IN FUNKTIONALISIERTEM SP2-HYBRIDISIERUNGSZUSTAND, IHR HERSTELLUNGSVERFAHREN UND IHRE ANWENDUNGEN, INSBESONDERE UM EINE OBERFLÄCHE ANTIBAKTERIELL ZU MACHEN
ASSEMBLY OF CARBON ATOMS IN FUNCTIONALISED SP2 HYBRIDISATION STATE, METHOD FOR PREPARING SAME AND USES THEREOF, IN PARTICULAR FOR MAKING A SURFACE ANTI-BACTERIAL

(30) Priorité: 29.07.2020 FR 2008040
(43) Date de publication de la demande: 02.02.2022
(73) Titulaire: Commissariat à l'Energie Atomique et aux Energies Alternatives, 75015 Paris (FR)
(72) Inventeur: ALAVA, Thomas, 38054 GRENOBLE (FR); KUMAR, Madhav, 38054 GRENOBLE (FR)
(74) Mandataire: Gevers & Orès

(56) Documents cités:
- CN-A- 109 125 708
- KR-A- 20150 143 174
- JASON A. MANN ET AL: "Preservation of Antibody Selectivity on Graphene by Conjugation to a Tripod Monolayer", ANGEWANDTE CHEMIE INTERNATIONAL EDITION, vol. 52, no. 11, 11 March 2013 (2013-03-11), pages 3177 - 3180, XP055098219, ISSN: 1433-7851, DOI: 10.1002/anie.201209149
- PANDEY ASHISH ET AL: "Graphene-interfaced electrical biosensor for label-free and sensitive detection of foodborne pathogenicE. coliO157:H7", BIOSENSORS AND BIOELECTRONICS, ELSEVIER SCIENCE LTD. UK, AMSTERDAM, NL, vol. 91, 16 December 2016 (2016-12-16), pages 225 - 231, XP029920732, ISSN: 0956-5663, DOI: 10.1016/J.BIOS.2016.12.041
- CHEN R J ET AL: "Noncovalent Sidewall Functionalization of Single-Walled Carbon Nanotubes for Protein Immobilization", JOURNAL OF THE AMERICAN CHEMICAL SOCIETY, AMERICAN CHEMICAL SOCIETY, US, vol. 123, 1 January 2001 (2001-01-01), pages 3838 - 3839, XP002350390, ISSN: 0002-7863, DOI: 10.1021/JA010172B
- BATTIGELLI ALESSIA ET AL: "Endowing carbon nanotubes with biological and biomedical properties by chemical modifications", ADVANCED DRUG DELIVERY REVIEWS, ELSEVIER, AMSTERDAM , NL, vol. 65, no. 15, 13 July 2013 (2013-07-13), pages 1899 - 1920, XP028791634, ISSN: 0169-409X, DOI: 10.1016/J.ADDR.2013.07.006

## Description

La présente invention concerne un ensemble d'atomes de carbone à l'état d'hybridation sp² fonctionnalisé par un anticorps antibactérien ou une lectine, et un peptide antibactérien, son procédé de préparation, et ses utilisations, notamment pour rendre une surface antibactérienne. La présente invention vise également un matériau recouvert d'un tel ensemble fonctionnalisé, ainsi que son procédé de préparation.

Outre les produits dits « désinfectants », et les usages que l'on en fait pour nettoyer ou même stériliser les sols, les mains, des surfaces diverses, il existe une autre approche pour éviter le développement bactérien, consistant à rendre directement les surfaces antibactériennes. Cette approche s'avère durable et peu coûteuse, car elle ne nécessite que des quantités de matière active très faibles. Ces traitements résistent généralement bien aux agressions extérieures et ont donc de nombreuses utilisations potentielles (bloc opératoire, agroalimentaire, habitations...).

Des ensembles d'atome de carbone à l'état d'hybridation sp², par exemple des flocons de graphène ou d'oxyde de graphène, ont un effet bactéricide avéré. Cependant, il a été montré que l'effet bactéricide des flocons de graphène ou d'oxyde de graphène, ne provenait que des bords des flocons. Les bords des flocons sont tellement rugueux qu'ils provoquent le déchirement de la membrane cellulaire des bactéries et donc la mort cellulaire de celles-ci. Ainsi, le pouvoir bactéricide d'une surface recouverte de flocon de graphène ou d'oxyde de graphène sera inversement proportionnel à la taille des flocons. Or, plus les flocons de graphène ou d'oxyde de graphène sont petits, plus ils sont toxiques, notamment en cas d'ingestion.

Les ensembles d'atomes de carbone à l'état d'hybridation sp² en couche continue, notamment le graphène en couche continue, sont à l'inverse extrêmement peu toxiques, mais aucun caractère antibactérien en provenance de la partie basale plane n'a été relevée.

Des fonctionnalisations chimiques de surfaces pour les rendre antibactériennes ont notamment été décrites, généralement à base de polymères, mais ces polymères ne sont pas transparents (ce qui peut être gênant pour certaines applications) et/ou ne s'adaptent pas à toutes les surfaces à traiter, et/ou ne confèrent à la surface traitée qu'un caractère bactéricide, alors que des surfaces aux propriétés bactériostatiques sont parfois préférées en fonction des applications visées.

Pandey Ashish et al. (Biosensors and Bioelectronics 2017, 91, 225-231) décrit la biofonctionnalisation de graphène par ancrage de molécules comprenant un pyrène et portant un anticorps anti-*E*. *coli* O157-H7.

Chen Robert J. et al. (J. Am. Chem. Soc. 2001, 123, 3838-3839) décrit la biofonctionnalisation de nanotubes de carbone par ancrage de molécules comprenant un pyrène et portant une protéine.

Battigelli Alessia et al. (Advanced Drug Delivery Reviews 2013, 65, 1899-1920) décrit l'immobilisation de protéines sur des nanotubes de carbone par conjugaison via des liaisons non covalents de type interactions π - π, notamment par utilisation de linkers de type acide 1-pyrènebutyrique N-hydroxysuccinimide ester et acide naphtalène-1-ylméthylphosphonique.L'invention a pour but de permettre la mise en oeuvre d'une méthode de fonctionnalisation d'une surface pour la rendre antibactérienne qui évite les inconvénients précités.

Ainsi, un but de l'invention est de fournir une méthode permettant un traitement antibactérien de tout type de surface, que ce soit en termes de nature physico-chimique et/ou de forme, de topographie.

Un autre but de l'invention est de fournir une méthode qui permet si nécessaire, en fonction de l'application visée, un réglage fin et aisé des propriétés antibactérienne de la surface traitée, par exemple un caractère bactéricide ou bactériostatique.

Un autre but de l'invention est de fournir une surface fonctionnalisée antibactérienne, dont la fonctionnalisation peut être transparente aux longueurs d'onde du visible.

Ainsi, selon un premier aspect, l'invention concerne un ensemble d'atomes de carbone à l'état d'hybridation sp², ledit ensemble étant en contact avec :
- une pluralité de composés de formule (I) suivante :

   (R-L₁-W)ₙ-V-L₂-X-Y (I),

   dans laquelle :
   n est égal à 1, 2, ou 3, notamment 3 ;
   V représente, pour tout n, -C(H)₃₋ₙ- ou encore:
      - lorsque n est égal à 2, -C(H)=, C étant dans ce cas lié à L₂ par une liaison double ;
      - lorsque n est égal à 1, -C=, C étant dans ce cas lié à L₂ par une liaison triple ;
   R est un hydrocarbure aromatique comprenant de 2 à 6 cycles aromatiques condensés, en particulier à chaque occurrence indépendamment choisi parmi les groupes pyrènyle, benzopyrènyle, anthracènyle, chrystènyle, coronènyle, corannulènyle, naphtyle, tétracènyle, pentacènyle, phénathrènyle, triphénylènyle et ovalènyle, plus particulièrement parmi les groupes pyrènyle, benzopyrènyle, anthracènyle ;
   L₁ un groupe C₁-C₁₂ alcane diyle ;
   W représente une liaison simple, un groupe arène diyle, un groupe hétéroarène diyle ou un groupe -O-Ar₁- où Ar₁ est un arène ou un hétéroarène ;
   L₂ est un groupe de formule (A) suivante -(L₂ₐ)ᵢ-(L_{2b})ⱼ- dans laquelle :
      i et j sont indépendamment l'un de l'autre choisis parmi 0 et 1, avec i + j = 1 ou 2 ;
      L₂ₐ est un groupe C₁-C₁₂ alcane diyle, C₂-C₁₂ alcène diyle ou C₂-C₁₂ alcyne diyle ;
      L_{2b} est un groupe arène diyle, un groupe hétéroarène diyle ou un groupe -O-Ar₂- où
      Ar₂ est un arène ou un hétéroarène ;
   X est un groupe -C(=O)N-, -C(=O)O- ou -C(=O)S- ;
   Y est un anticorps antibactérien ou une lectine ;
- une pluralité de composés de formule (II) suivante :

   (R-L₁-W)ₙ-V-L₂-X-Z (II),

   dans laquelle :
   n, R, L₁, W, V, L₂ et X sont tels que définis plus haut ;
   Z est un peptide antibactérien.

Ainsi, dans les deux formules (I) et (II), C représente un atome de carbone tétravalent dans le cas où V représente -C(H)₃₋ₙ- (-C-, -CH-, ou -CH₂- suivant la valeur de n); un atome de carbone trivalent lorsque V représente -C(H)= ; ou un atome de carbone divalent lorsque V représente -C=.

Par « en contact », on entend notamment que les hydrocarbures aromatiques (R) des composés de formule (I) et (II) forment avec l'ensemble d'atomes de carbone à l'état d'hybridation sp² des liaisons de type empilement-Pi. Ces liaisons, appelées également empilement π- π ou Pi-stacking en anglais, sont des liaisons non covalentes, parmi les plus fortes des liaisons non covalentes.

Selon un mode de réalisation particulier, ledit ensemble d'atomes de carbone à l'état d'hybridation sp² est choisi parmi le graphène, l'oxyde de graphène, le graphite, les nanotubes de carbones, les fullerènes et les fullerites, ledit ensemble étant en particulier du graphène, plus particulièrement sous la forme de feuille ou de flocons.

Par « feuille », on entend notamment du graphène, en particulier mono-couche, dont deux des dimensions sont supérieures à 1 mm.

Par « flocon », on entend notamment du graphène, en particulier ayant de 1 à 10 couches, par exemple mono-couche, dont l'une voire deux des dimensions est(sont) inférieure(s) ou égale(s) à 1 mm, en particulier 500µm, 100 µm ou 10 µm.

Selon un mode de réalisation particulier, l'invention concerne un ensemble d'atomes de carbone à l'état d'hybridation sp², ledit ensemble étant en contact avec :
- une pluralité de composés de formule (Ia) suivante :

   (Py-L₁-W)₃-C-L₂-X-Y (Ia),

   dans laquelle :
   Py est un groupe pyrènyle ;
   L₁ un groupe C₁-C₁₂ alcane diyle ;
   W représente une liaison simple, un groupe arène diyle, un groupe hétéroarène diyle ou un groupe -O-Ar₁- où Ar₁ est un arène ou un hétéroarène ;
   L₂ est un groupe de formule (A) suivante -(L₂ₐ)ᵢ-(L_{2b})ⱼ- dans laquelle :
      i et j sont indépendamment l'un de l'autre choisis parmi 0 et 1, avec i + j = 1 ou 2 ;
      L₂ₐ est un groupe C₁-C₁₂ alcane diyle, C₂-C₁₂ alcène diyle ou C₂-C₁₂ alcyne diyle ;
      L_{2b} est un groupe arène diyle, un groupe hétéroarène diyle ou un groupe -O-Ar₂- où
      Ar₂ est un arène ou un hétéroarène ;
   X est un groupe -C(=O)N-, -C(=O)O- ou -C(=O)S- ;
   Y est un anticorps antibactérien ; et
- une pluralité de composés de formule (IIa) suivante :

   (Py-L₁-W)₃-C-L₂-X-Z (IIa),

   dans laquelle :
   Py, L₁, W, L₂ et X sont tels que définis plus haut ;
   Z est un peptide antibactérien.

Selon un mode de réalisation particulier, ledit ensemble d'atomes de carbone à l'état d'hybridation sp² est supporté par un substrat, notamment choisi parmi les métaux, les oxydes métalliques, les verres et les polymères.

Selon un mode de réalisation particulier, les groupes R, L₁, W, V, L₂ et éventuellement X du composé (I) sont identiques à ceux du composé de formule (II).

Selon un mode de réalisation particulier, R est le 1-pyrènyle ou le 2-pyrènyle, en particulier le 1-pyrènyle.

Selon un mode de réalisation particulier, L₁ un groupe C₂-C₈ alcane diyle, notamment le butane diyle.

Selon un mode de réalisation particulier, W représente un groupe -O-Ar₁- où Ar₁ est un arène ou un hétéroarène, en particulier un benzène, plus particulièrement un benzène substitué en para.

Selon un mode de réalisation particulier, i et j sont tous deux égaux à 1.

Selon un mode de réalisation particulier, L₂ₐ est un groupe C₂-C₁₂ alcyne diyle, en particulier -C=C-.

Selon un mode de réalisation particulier, L_{2b} est un groupe arène diyle, en particulier un benzène diyle, plus particulièrement un benzène diyle substitué en para.

Selon un mode de réalisation particulier, R, L₁, W, V, L₂ sont tels que définis par la formule suivante :

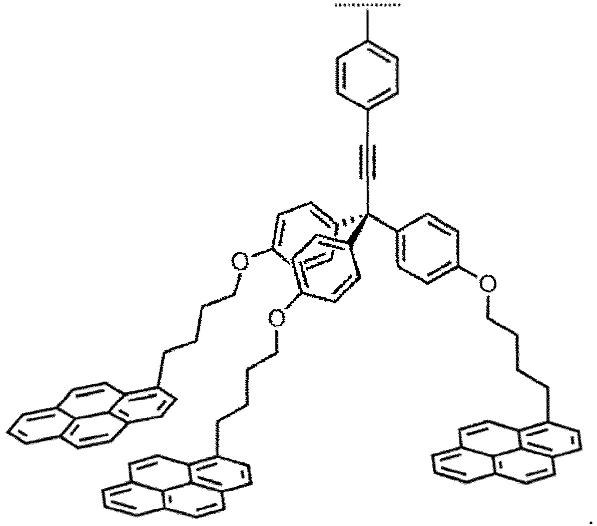

Selon un mode de réalisation particulier, Y est choisi parmi les anticorps anti Escherichia coli, les anticorps anti bactéries gram positives, les anticorps anti bactéries gram négatives, Y étant notamment un anticorps anti Escherichia coli.

Les anticorps anti Escherichia coli sont bien connus de l'homme du métier. Il s'agit par exemple d'anticorps polyclonaux, notamment de chèvre ou de lapin, en particulier ceux reconnaissant de nombreux sérotypes antigéniques « O » et « K » d' Escherichia coli, notamment ceux commercialisés par LifeSpan BioSciences (par exemple sous la référence LS-C58854) ou par Abcam (par exemple sous la référence ab137967).

Il peut également s'agir d'anticorps anti Escherichia coli, de préférence monoclonaux, notamment de souris, dirigés spécifiquement contre des souches particulières d' E. coli, par exemple des souches particulièrement pathogènes comme la souche 0157. C'est notamment le cas des anticorps anti E Coli 0157 commercialisés par MyBioSource (par exemple sous la référence MBS568193).

Les anticorps anti bactéries gram positives et les anticorps anti bactéries gram négatives sont également bien connus de l'homme du métier. Il peut notamment s'agir d'anticorps monoclonaux dirigés contre les endotoxines des bactéries à Gram négatif, notamment ceux commercialisés par ThermoFisher Scientific (par exemple sous la référence MA1-10685).

Selon un mode de réalisation particulier, Y est choisi parmi les lectines, en particulier celles se liant à des sucres des parois bactériennes, plus particulièrement les sucres présents sur la paroi des bactéries Gram positif.

Selon un mode de réalisation particulier, Y est la concanavaline A. Ce composé est notamment commercialisé par Sigma-Aldrich (par exemple sous la référence C5275).

Selon un mode de réalisation particulier, Z est choisi parmi les cécropines, les défensines, les magainines et les dermaseptines, Z étant notamment une cécropine.

Selon un mode de réalisation plus particulier, Z est choisi parmi les cécropines, notamment la cécropine A (SEQ ID NO :1), la cécropine B (SEQ ID NO :2), la cécropine P1 (SEQ ID NO :3), la cécropine 1 (SEQ ID NO :4) et la cécropine de *Bombyx mori* (SEQ ID NO :5), les défensines, notamment la défensine 6 (SEQ ID NO :6) et la défensine 5 (SEQ ID NO :7), les magainines, notamment la magainine B2 (SEQ ID NO :8) et la magainine R2 (SEQ ID NO :9) et les dermaseptines, notamment la dermaseptine H3 (SEQ ID NO : 10) et la dermaseptine S1 (SEQ ID NO :11).

Les cécropines A, B et P1 sont notamment commercialisées par Sigma Aldrich.

Selon un mode de réalisation particulier, la densité en composés de formule (I) et (II) à la surface de l'ensemble d'atomes de carbone à l'état d'hybridation sp² est comprise d'environ 1 de ces composés par surface de 300 nm² à environ 1 de ces composés par surface de 2 nm², notamment environ 1 composé par surface d'environ 2,7 nm².

Selon un mode de réalisation particulier, le rapport molaire de la quantité de composés de formule (I) sur celle de composés de formule (II) est compris de 0,01 à 100, notamment de 0,1 à 10, en particulier de 0,33 à 1,5, plus particulièrement de 0,33 à 0,66 ou de 0,66 à 1,5, par exemple environ 0,5 ou environ 1,0.

Selon un mode de réalisation plus particulier, l'ensemble d'atomes de carbone à l'état d'hybridation sp² est du graphène sous la forme de feuille, le rapport molaire de la quantité de composés de formule (I) sur celle de composés de formule (II) étant compris de 0,66 à 1,5, par exemple environ 1,0.

Selon un mode de réalisation plus particulier, l'ensemble d'atomes de carbone à l'état d'hybridation sp² est du graphène sous la forme de flocons, le rapport molaire de la quantité de composés de formule (I) sur celle de composés de formule (II) étant compris de 0,33 à 0,66, par exemple environ 0,5.

Selon un autre aspect, l'invention concerne également un procédé de préparation d'un ensemble d'atomes de carbone à l'état d'hybridation sp² fonctionnalisé tel que défini précédemment, comprenant les étapes suivantes :
(i) Mise en contact d'un ensemble d'atomes de carbone à l'état d'hybridation sp² avec un composé de formule (III) suivante :

   (R-L₁-W)ₙ-V-L₂-X-Q (III),

   dans laquelle :
   R, L₁, W, V et L₂ sont définis comme précédemment;
   X-Q est un groupe choisi parmi -C(=O)O-*N*-succinimidyle, -C(=O)-halogènure, en particulier -C(=O)-Cl, -C(=O)-N₃, -C(=O)-O-*N*-imidazolyle, -C(=O)-O-C(=O)-R', avec R' étant un C₁-C₁₂ alkyle linéaire, ramifié ou cyclique, -C(=O)-O-catécholborane, -C(=O)-O-benzotriazole, en particulier -C(=O)O-*N*-succinimidyle,
   pour obtenir un ensemble d'atomes de carbone à l'état d'hybridation sp² activé ;
(ii) Mise en contact de l'ensemble d'atomes de carbone à l'état d'hybridation sp² activé tel qu'obtenu à l'étape précédente avec une composition comprenant un anticorps antibactérien ou une lectine et un peptide antibactérien, pour obtenir un ensemble d'atomes de carbone à l'état d'hybridation sp² fonctionnalisé.

De façon alternative, la mise en contact de l'étape (ii) peut se faire de façon séquentielle, c'est-à-dire comprendre une première mise en contact avec une composition comprenant un anticorps antibactérien ou une lectine puis une deuxième mise en contact avec une composition comprenant un peptide antibactérien, ou une première mise en contact avec une composition comprenant un peptide antibactérien puis une deuxième mise en contact avec une composition comprenant un anticorps antibactérien ou une lectine.

Ce procédé permet le traitement antibactérien de tout ensemble d'atomes de carbone à l'état d'hybridation sp², notamment de graphène, en particulier le graphène des biocapteurs au graphène.

Tous les modes de réalisation définis plus haut à propos de l'ensemble d'atomes de carbone à l'état d'hybridation sp² (fonctionnalisé) s'appliquent également ici, seuls ou en combinaison.

Selon un autre aspect, l'invention concerne également l'utilisation d'un ensemble d'atomes de carbone à l'état d'hybridation sp² tel que défini plus haut, pour rendre une surface antibactérienne, en particulier bactéricide ou bactériostatique.

Tous les modes de réalisation définis plus haut à propos de l'ensemble d'atomes de carbone à l'état d'hybridation sp² s'appliquent également ici, seuls ou en combinaison.

Selon un autre aspect, l'invention concerne également un matériau comprenant un substrat dont tout ou partie de la surface est recouverte d'un ensemble d'atomes de carbone à l'état d'hybridation sp² fonctionnalisé (c'est-à-dire au contact de composés de formule (I) et (II)) tel que défini précédemment.

Tous les modes de réalisation définis plus haut à propos de l'ensemble d'atomes de carbone à l'état d'hybridation sp² s'appliquent également ici, seuls ou en combinaison.

Selon un mode de réalisation particulier, le substrat est choisi parmi les métaux, les oxydes métalliques, les verres et les polymères.

Selon un autre aspect, l'invention concerne également un procédé de préparation d'un matériau tel que défini plus haut, comprenant les étapes suivantes :
(i) Mise en contact d'un ensemble d'atomes de carbone à l'état d'hybridation sp² avec un composé de formule (III) suivante :

   (R-_{L}1-W)ₙ-V-L₂-X-Q (III),

   dans laquelle :
   R, L₁, W, V et L₂ sont tels que définis plus haut;
   X-Q est un groupe choisi parmi -C(=O)O-*N*-succinimidyle, -C(=O)-halogènure, en particulier -C(=O)-Cl, -C(=O)-N₃, -C(=O)-O-*N*-imidazolyle, -C(=O)-O-C(=O)-R', avec R' étant un C₁-C₁₂ alkyle linéaire, ramifié ou cyclique, -C(=O)-O-catécholborane, -C(=O)-O-benzotriazole, en particulier -C(=O)O-N-succinimidyle,
   pour obtenir ensemble d'atomes de carbone à l'état d'hybridation sp² activé;
(ii) Mise en contact de l'ensemble d'atomes de carbone à l'état d'hybridation sp² activé tel qu'obtenu à l'étape précédente avec une composition comprenant un anticorps antibactérien ou une lectine et un peptide antibactérien, pour obtenir un ensemble d'atomes de carbone à l'état d'hybridation sp² fonctionnalisé ;
(iii) Dépôt de l'ensemble d'atomes de carbone à l'état d'hybridation sp² fonctionnalisé tel qu'obtenu à l'étape précédente sur la surface d'un support pour obtenir ledit matériau.

Tous les modes de réalisation définis plus haut à propos de l'ensemble d'atomes de carbone à l'état d'hybridation sp² et d'un procédé s'appliquent également ici, seuls ou en combinaison.

Selon un mode de réalisation particulier, la mise en contact de l'étape (i) se fait à l'aide d'une solution du composé de formule (III) dans un solvant.

Selon un mode de réalisation plus particulier, ledit solvant est choisi parmi les solvants apolaires aprotiques, notamment le tétrahydrofurane, le n-hexane, le cyclohexane, le 1,4-dioxane, le toluène, le diéthyl éther, l'acétate d'éthyle, le dichlorométhane, en particulier le tétrahydrofurane.

Selon un mode de réalisation plus particulier, le composé de formule (III) est présent dans la solution à une concentration comprise de 1 nM à 100 µM, par exemple à environ 1µM.

Selon un mode de réalisation particulier, la mise en contact de l'étape (i) se fait pendant une durée de 10 secondes à 300 secondes, par exemple environ 60 secondes.

Selon un mode de réalisation particulier, l'étape (i) est suivie, préalablement à l'étape (ii) d'une étape de lavage de l'ensemble d'atomes de carbone à l'état d'hybridation sp² activé, en particulier à l'eau.

Selon un mode de réalisation particulier, la mise en contact de l'étape (ii) se fait à l'aide d'une solution de l'anticorps antibactérien ou de la lectine et du peptide antibactérien dans un solvant.

Selon un mode de réalisation plus particulier, ledit solvant est choisi parmi les solutions salines tamponnées au phosphate, notamment le PBS 1X.

Selon un mode de réalisation plus particulier, l'anticorps antibactérien ou la lectine et le peptide antibactérien sont présents dans la solution à une concentration totale comprise de 10 pM à 10 µM, par exemple à environ 10 nM.

Selon un mode de réalisation particulier, la mise en contact de l'étape (ii) se fait pendant une durée de 10 minutes à 100 minutes, par exemple environ 30 minutes.

Selon un mode de réalisation particulier, l'étape (ii) est suivie, préalablement à l'étape (iii) d'une étape de lavage de l'ensemble d'atomes de carbone à l'état d'hybridation sp² fonctionnalisé, en particulier à l'aide d'une solution saline tamponnée au phosphate, notamment le PBS 1X.

Selon un mode de réalisation particulier, le dépôt de l'ensemble d'atomes de carbone à l'état d'hybridation sp² fonctionnalisé de l'étape (iii) se fait par transfert d'un substrat vers ladite surface, notamment d'un substrat cuivre, en particulier par voie humide (« wet transfert »), par exemple selon Won Suk et al. (ACS Nano 2011, 5(9), 6916-6924), ou par voie sèche (« dry transfert »), par exemple selon Vaziri *et al.* (PDMS-supported graphene transfer using intermediary polymer layers, 2014 44th European Solid State Device Research Conférence (ESSDERC), 309-312) ou Caldwell et al. (ACS Nano 2010, 4(2), 1108-1114).

A titre d'exemple pour le graphène, lors d'un transfert par voie humide, la couche de cuivre ayant servi à faire croître le graphène peut notamment être dissoute à la surface d'un bain de gravure du cuivre (liquide) puis le graphène est mis en contact avec la surface dudit support pour obtenir ledit matériau.

Egalement à titre d'exemple pour le graphène, lors d'un transfert par voie sèche, le graphène peut notamment être détaché du substrat cuivre de croissance par ajout d'un polymère (par exemple le parylène PDMS), puis l'ensemble polymère+graphène est apposé sur la surface désirée par action mécanique (peeling mécanique). Le polymère étant ensuite retiré.

Selon un autre aspect, l'invention concerne un procédé de préparation d'un matériau tel que défini précédemment, comprenant les étapes suivantes :
(i) Mise en contact d'un ensemble d'atomes de carbone à l'état d'hybridation sp² porté par la surface d'un matériau avec un composé de formule (III) suivante :

   (R-L₁-W)ₙ-V-L₂-X-Q (III),

   dans laquelle :
   R, L₁, W, V et L₂ sont tels que définis plus haut;
   X-Q est un groupe choisi parmi -C(=O)O-*N*-succinimidyle, -C(=O)-halogènure, en particulier -C(=O)-Cl, -C(=O)-N₃, -C(=O)-O-*N*-imidazolyle, -C(=O)-O-C(=O)-R', avec R' étant un C₁-C₁₂ alkyle linéaire, ramifié ou cyclique, -C(=O)-O-catécholborane, -C(=O)-O-benzotriazole, en particulier -C(=O)O-*N*-succinimidyle,
   pour obtenir un matériau activé ;
(ii) Mise en contact du matériau activé tel qu'obtenu à l'étape précédente avec une composition comprenant un anticorps antibactérien ou une lectine et un peptide antibactérien, pour obtenir ledit matériau, fonctionnalisé.

Ce procédé permet ainsi de traiter tout matériau portant un ensemble d'atomes de carbone à l'état d'hybridation sp², par exemple du graphène, et les dispositifs comprenant ledit matériau.

Par exemple, ce procédé permet le traitement antibactérien de biocapteurs au graphène.

Tous les modes de réalisation définis plus haut à propos de l'ensemble d'atomes de carbone à l'état d'hybridation sp² et d'un procédé s'appliquent également ici, seuls ou en combinaison.

Selon un mode de réalisation particulier, l'ensemble d'atomes de carbone à l'état d'hybridation sp² porté par la surface du matériau est préalablement déposé sur ladite surface au moyen de toute technique bien connue de l'homme du métier.

Dans le cas du graphène sous forme de feuille, ladite feuille peut notamment être obtenue par toute technique connue de l'homme du métier, par exemple par voie chimique en phase vapeur (CVD), notamment sur substrat de cuivre, en particulier comme décrit par Li et al. (Journal of the American Chemical Society 2011, 133, 2816-2819), puis par transfert de la feuille ainsi obtenue sur ladite surface, par toute technique connue de l'homme du métier, par exemple par voie liquide, en particulier comme décrit par Reina et al. (Journal of the American Chemical Society 2011, 133, 17614-17617).

### Définitions

Tel qu'on l'utilise dans la présente description, le terme « environ » se réfère à un intervalle de valeurs de ± 10 % d'une valeur spécifique. A titre d'exemple, l'expression « environ 20 » comprend les valeurs de 20 ± 10 %, soit les valeurs de 18 à 22.

Au sens de la présente description, les pourcentages se réfèrent à des pourcentages en masse par rapport à la masse totale de la formulation, sauf indication contraire.

Tel qu'on l'entend ici, les plages de valeur sous forme de « x-y » ou « de x à y » ou « entre x et y » incluent les bornes x et y ainsi que les entiers compris entre ces bornes. A titre d'exemple, « 1-5 », ou « de 1 à 5 » ou « entre 1 et 5 » désignent les entiers 1, 2, 3, 4 et 5. Les modes de réalisations préférés incluent chaque entier pris individuellement dans la plage de valeur, ainsi que toute sous-combinaison de ces entiers. A titre d'exemple, les valeurs préférées pour « 1-5 » peuvent comprendre les entiers 1, 2, 3, 4, 5, 1-2, 1-3, 1-4, 1-5, 2-3, 2-4, 2-5, etc.

Tel qu'il est utilisé ici, le terme "alkyle" désigne un groupe alkyle à chaîne linéaire ou ramifiée ayant le nombre d'atomes de carbone indiqué avant ledit terme, notamment 1 à 8 atomes de carbone, tel que méthyle, éthyle, propyle, isopropyle, butyle, isobutyle, sec-butyle, tert-butyle, pentyle, isoamyle, néopentyle, 1-éthylpropyle, 3-méthylpentyle, 2,2-diméthylbutyle, 2,3-diméthylbutyle, hexyle, octyle, etc. Ainsi, une expression telle que "alkyle en C1-C4" désigne un radical alkyle contenant de 1 à 4 atomes de carbone. Il en est de même pour le terme « alcane ». Tel qu'utilisé ici, le terme "arène" désigne un système cyclique aromatique hydrocarboné mono- ou bicyclique, substitué ou non substitué, ayant 6 à 10 atomes de carbone dans le cycle. Les exemples incluent le benzène et le naphtalène. Les arènes préférés comprennent le benzène et le naphtalène non substitués ou substitués. Sont inclus dans la définition d'"arène" les systèmes cycliques condensés, y compris, par exemple, les systèmes cycliques dans lesquels un cycle aromatique est condensé à un cycle cycloalkyle. Les exemples de tels systèmes cycliques condensés comprennent, par exemple, l'indane, l'indène et le tétrahydronaphtalène.

Tel qu'il est utilisé ici, le terme "hétéroarène" désigne un système aromatique cyclique contenant 5 à 10 atomes de carbone dans lequel un ou plusieurs atomes de carbone du cycle sont remplacés par au moins un hétéroatome tel que -O-, -N- ou -S-. Les exemples d'hétéroarènes comprennent pyrrole, furane, thiophène, pyrazole, imidazole, thiazole, isothiazole, isoxazole, oxazole, oxathiol, oxadiazole, triazole, oxatriazole, furazane, tétrazole, pyridine, pyrazine, pyrimidine, pyridazine, triazine, indole, isoindole, indazole, benzofurane, isobenzofurane, purine, quinazoline, quinoline, isoquinoline, benzoimidazole, benzothiazole, benzothiophène, thianaphtène, benzoxazole, benzisoxazole, cinnoline, phtalazine, naphtyridine et quinoxaline. Sont inclus dans la définition d'"hétéroarène" les systèmes cycliques condensés, y compris, par exemple, les systèmes cycliques dans lesquels un cycle aromatique est condensé à un cycle hétérocycloalkyle. Les exemples de tels systèmes cycliques fusionnés comprennent, par exemple, le phtalamide, l'anhydride phtalique, l'indoline, l'isoindoline, la tétrahydroisoquinoline, le chromane, l'isochromane, le chromène et l'isochromène.

| | |
|---|---|
| SEQ ID NO :1 | Lys Trp Lys Leu Phe Lys Lys Ile Glu Lys Val Gly Gln Asn Ile Arg Asp Gly Ile Ile Lys Ala Gly Pro Ala Val Ala Val Val Gly Gln Ala Thr Gln Ile Ala Lys |
| SEQ ID NO :2 | Lys Trp Lys Val Phe Lys Lys Ile Glu Lys Met Gly Arg Asn Ile Arg Asn Gly Ile Val Lys Ala Gly Pro Ala Ile Ala Val Leu Gly Glu Ala Lys Ala Leu |
| SEQ ID NO :3 | Ser Trp Leu Ser Lys Thr Ala Lys Lys Leu Glu Asn Ser Ala Lys Lys rg Ile Ser Glu Gly Ile Ala Ile Ala Ile Gln Gly Gly Pro Arg |
| SEQ ID NO :4 | Met Asn Phe Asn Lys Val Phe Ile Leu Val Ala Ile Val Ile Ala Ile Phe Ala Gly Gln Thr Glu Ala Gly Trp Leu Lys Lys Ile Gly Lys Lys Ile Glu Arg Val Gly Gln His Thr Arg Asp Ala Thr Ile Gln Thr Ile Ala Val Ala Gln Gln Ala Ala Asn Val Ala Ala Thr Ala Arg Gly |
| SEQ ID NO :5 | Arg Trp Lys Ile Phe Lys Lys Ile Glu Lys Val Gly Gln Asn Ile Arg Asp Gly Ile Val Lys Ala Gly Pro Ala Val Ala Val Val Gly Gln Ala Ala Thr Ile |
| SEQ ID NO :6 | Met Arg Thr Leu Thr Ile Leu Thr Ala Val Leu Leu Val Ala Leu Gln Ala Lys Ala Glu Pro Leu Gln Ala Glu Asp Asp Pro Leu Gln Ala Lys Ala |
| | Tyr Glu Ala Asp Ala Gln Glu Gln Arg Gly Ala Asn Asp Gln Asp Phe Ala Val Ser Phe Ala Glu Asp Ala Ser Ser Ser Leu Arg Ala Leu Gly Ser Thr Arg Ala Phe Thr Cys His Cys Arg Arg Ser Cys Tyr Ser Thr Glu Tyr Ser Tyr Gly Thr Cys Thr Val Met Gly Ile Asn His Arg Phe Cys Cys Leu |
| SEQ ID NO :7 | Met Arg Thr Ile Ala Ile Leu Ala Ala Ile Leu Leu Val Ala Leu Gln Ala Gln Ala Glu Ser Leu Gln Glu Arg Ala Asp Glu Ala Thr Thr Gln Lys Gln Ser Gly Glu Asp Asn Gln Asp Leu Ala Ile Ser Phe Ala Gly Asn Gly Leu Ser Ala Leu Arg Thr Ser Gly Ser Gln Ala Arg Ala Thr Cys Tyr Cys Arg Thr Gly Arg Cys Ala Thr Arg Glu Ser Leu Ser Gly Val Cys Glu Ile Ser Gly Arg Leu Tyr Arg Leu Cys Cys Arg |
| SEQ ID NO :8 | Gly Ile Gly Lys Phe Leu His Ser Ala Gly Lys Phe Gly Lys Ala Phe Leu Gly Glu Val Met Lys Ser |
| SEQ ID NO :9 | Gly Ile Lys Glu Phe Ala His Ser Leu Gly Lys Phe Gly Lys Ala Phe Val Gly Gly Ile Leu Asn Gln |
| SEQ ID NO :10 | Met Ala Phe Leu Lys Lys Ser Leu Phe Leu Val Leu Phe Leu Gly Met Val Ser Leu Ser Ile Cys Glu Glu Glu Lys Arg Glu Asn Glu Asp Glu Glu Leu Gln Glu Asp Asp Glu Gln Ser Glu Met Lys Arg Gly Leu Trp Ser Thr Ile Lys Asn Val Gly Lys Glu Ala Ala Ile Ala Ala Gly Lys Ala Ala Leu Gly Ala Leu |
| SEQ ID NO :11 | Met Asp Ile Leu Lys Lys Ser Leu Phe Leu Val Leu Phe Leu Gly Leu Val Ser Leu Ser Ile Cys |
| | Glu Glu Glu Lys Arg Glu Asn Glu Asp Glu Glu Lys Gln Glu Asp Asp Glu Gln Ser Glu Met Lys Arg Ala Leu Trp Lys Thr Met Leu Lys Lys Leu Gly Thr Met Ala Leu His Ala Gly Lys Ala Ala Leu Gly Ala Ala Ala Asp Thr Ile Ser Gln Gly Thr Gln |

### FIGURES

La figure 1 montre des images optiques relatives à la croissance de E. coli en fonction du temps, selon l'exemple 2, avec une surface telle que le ratio A:B est de 0,35:0,65 (7:13).
La figure 2 montre des images optiques relatives à la croissance de E. coli en fonction du temps, selon l'exemple 2, avec une surface telle que le ratio A:B est de 0,5:0,5 (1:1).

### EXEMPLES

### Exemple 1 : préparation d'un ensemble d'atomes de carbone à l'état d'hybridation sp² fonctionnalisé par un anticorps antibactérien ou une lectine et un peptide antibactérien

Un ensemble d'atomes de carbone à l'état d'hybridation sp², par exemple une couche de graphène, est synthétisé selon toute technique bien connue de l'homme du métier, notamment par CVD, puis optionnellement déposée sur une surface à traiter par toute technique bien connue de l'homme du métier, notamment par une méthode de transfert par voie liquide.

La couche de graphène est trempée dans une solution à 1 µM dans le tétrahydrofurane (THF) d'un tripode de formule suivante (tel que décrit par exemple par Mann et al., Angewandte Chemie International Edition 2013, 52, 3177-3180): 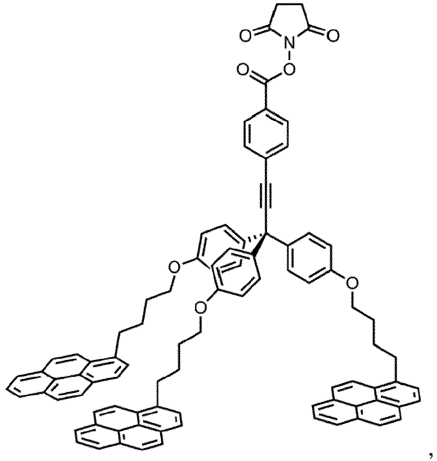 et ce, pendant une minute. Suite à quoi l'échantillon est trempé dans l'eau afin de laver l'excès de THF. On obtient ainsi une monocouche de tripodes à la surface du graphène avec une densité d'environ 1 molécule de tripode par 2,7 nm².

L'échantillon ainsi obtenu est incubé dans une solution de PBS 1X. Puis sont ajoutés dans cette solution des anticorps anti-Escherichia coli (qui ont la capacité de lier spécifiquement des bactéries de type Escherichia coli) tels que les anticorps LifeSpan BioSciences LS-C58854, Abcam ab137967 ou MyBioSource MBS568193, ainsi que des molécules de cécropines (qui ont la particularité de créer des trous dans la membrane cellulaire de bactéries et de provoquer leur mort cellulaire), comme la cécropine A, la cécropine B ou la cécropine P1 commercialisées par Sigma Aldrich (sous la référence C6830 pour la cécropine A), dans des proportions molaires précises, qui sont notamment discutées dans l'exemple suivant.

Le tripode est conçu de telle manière que l'ester N-Hydroxysuccinimide présent au sommet de la molécule va engager des liaisons covalentes avec tout type de peptides ou protéines.

Ainsi en injectant la solution anticorps : cécropine dans un mélange stoechiométrique A:B, ces molécules vont s'attacher de façon covalente une à une au sommet d'un tripode. Au bout de 30 minutes d'incubation, il y a à la surface du graphène une proportion A:B d'anticorps et de cécropine.

Suite à la déposition de ces molécules, les molécules non greffées à un tripode, sont rincés avec une solution fraiche de PBS1X (solution tampon) pendant 5 min.

### Exemple 2 : évaluation du caractère antibactérien d'un ensemble d'atomes de carbone à l'état d'hybridation sp² fonctionnalisé selon l'invention

### Protocole

Une couche de graphène par exemple produite par CVD (Li et al., Journal of the American Chemical Society 2011, 133, 2816-2819) est déposée (par exemple selon Reina et al. Journal of the American Chemical Society 2011, 133, 17614-17617) sur la surface d'une lame en verre de microscope.

Cette couche de graphène est fonctionnalisée selon l'invention, en particulier selon l'exemple 1. Puis, des bactéries de type Escherichia coli K12, qui ont été modifiées avec un plasmide qui leur permet de synthétiser en plus de leur métabolisme la protéine fluorescente Green Fluorescent Protein (ce qui permet d'observer les bactéries par microscopie en fluorescence), sont incubées à la surface pendant 30 min.

Les bactéries qui ne sont pas attachés à la surface sont ensuite rincées grâce à une solution de PBS1X pendant 5 min. Ensuite, on verse une solution nutritive pour bactéries (solution Luria Bertani, également connue sous le nom de milieu de culture LB). La température est maintenue à 37°C. Les bactéries attachées à la surface du graphène sont donc ainsi, surface antibactérienne mise à part, dans des conditions de croissance et de prolifération exponentielle.

### Résultats

Un témoin hors invention (surface avec un ratio A:B (anticorps :peptide antimicrobien) de 1:0) montre une croissance exponentielle des bactéries a la surface qui se mettent à former un biofilm.

### Ratio A:B compris de 0,25 : 0,75 à 0,40 : 0,60, notamment de 0,35 :0,65 (7:13)

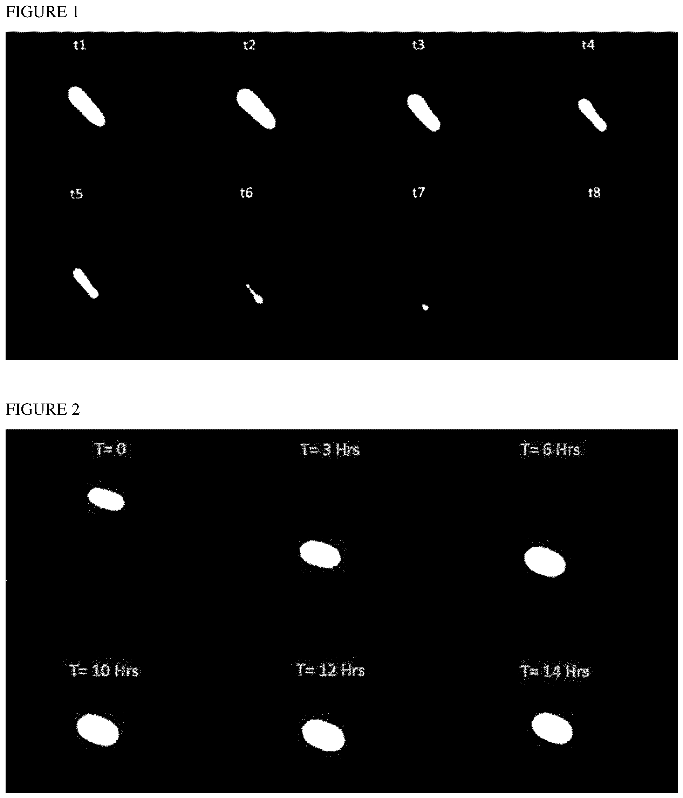

Avec un tel ratio, il est observé que la couche fonctionnalisée de l'invention provoque non seulement l'arrêt de la croissance des bactéries à la surface mais également leur mort cellulaire. Ceci est confirmé par l'analyse du signal de fluorescence au cours du temps (figure 1). Ce signal décroît, ce qui signifie que la membrane cellulaire est endommagée à un point tel que les protéines de GFP sortent de la bactérie qui est donc considérée comme morte (ou lysée).

Ainsi un tel ratio permet l'obtention d'une surface bactéricide.

### Ratio A:B compris de 0,40 : 0,60 à 0,60 : 0,40, notamment de 0,5:0,5 (1:1)

Avec un tel ratio, il est observé que la couche fonctionnalisée de l'invention provoque l'arrêt de la croissance des bactéries à la surface mais que les bactéries ne sont pas mortes. En effet, le signal lié à l'intensité de fluorescence GFP totale est constant dans le temps **(****figure 2****).**

Ainsi, avec ce ratio, la couche a toujours une propriété de limitation de la croissance de biofilms mais les bactéries ne sont plus tuées. Il s'agit donc d'un effet bactériostatique.

## Revendications

1. Ensemble d'atomes de carbone à l'état d'hybridation sp² fonctionnalisé, ledit ensemble d'atomes de carbone à l'état d'hybridation sp² étant en contact avec :
- une pluralité de composés de formule (I) suivante :
(R-L₁-W)ₙ-V-L₂-X-Y (I),
dans laquelle :
n est égal à 1, 2, ou 3, notamment 3 ;
V représente, pour tout n, -C(H)₃₋ₙ- ou encore:
- lorsque n est égal à 2, -C(H)=, C étant dans ce cas lié à L₂ par une liaison double ;
- lorsque n est égal à 1, -C=, C étant dans ce cas lié à L₂ par une liaison triple ;
R est un hydrocarbure aromatique comprenant de 2 à 6 cycles aromatiques condensés;
L₁ un groupe C₁-C₁₂ alcane diyle ;
W représente une liaison simple, un groupe arène diyle, un groupe hétéroarène diyle ou un groupe -O-Ar₁- où Ar₁ est un arène ou un hétéroarène ;
L₂ est un groupe de formule (A) suivante -(L₂ₐ)ᵢ-(L_{2b})ⱼ- dans laquelle :
i et j sont indépendamment l'un de l'autre choisis parmi 0 et 1, avec i + j = 1 ou 2 ;
L₂ₐ est un groupe C₁-C₁₂ alcane diyle, C₂-C₁₂ alcène diyle ou C₂-C₁₂ alcyne diyle ;
L_{2b} est un groupe arène diyle, un groupe hétéroarène diyle ou un groupe -O-Ar₂- où
Ar₂ est un arène ou un hétéroarène ;
X est un groupe -C(=O)N-, -C(=O)O- ou -C(=O)S- ;
Y est un anticorps antibactérien ou une lectine;
et
- une pluralité de composés de formule (II) suivante :
(R-L₁-W)ₙ-V-L₂-X-Z (II),
dans laquelle :
R, L₁, W, V, L₂ et X sont tels que définis plus haut ;
Z est un peptide antibactérien.

2. Ensemble selon la revendication 1, dans lequel ledit ensemble d'atomes de carbone à l'état d'hybridation sp² est choisi parmi le graphène, l'oxyde de graphène, le graphite, les nanotubes de carbones, les fullerènes et les fullerites, ledit ensemble étant en particulier du graphène, plus particulièrement sous la forme de feuille ou de flocons.

3. Ensemble selon l'une quelconque des revendications précédentes, dans lequel :
- R est le 1-pyrènyle ou le 2-pyrènyle, en particulier le 1-pyrènyle ;
- L₁ un groupe C₂-C₈ alcane diyle ; et/ou
- W représente un groupe -O-Ar₁- où Ar₁ est un arène ou un hétéroarène, en particulier un benzène ; et/ou
- i et j sont égaux à 1 ; et/ou
- L₂ₐ est un groupe C₂-C₁₂ alcyne diyle, en particulier -C=C- ; et/ou
- L_{2b} est un groupe arène diyle, en particulier un benzène diyle ; et/ou
- R, L₁, W, V et L₂ sont tels que définis par la formule suivante :

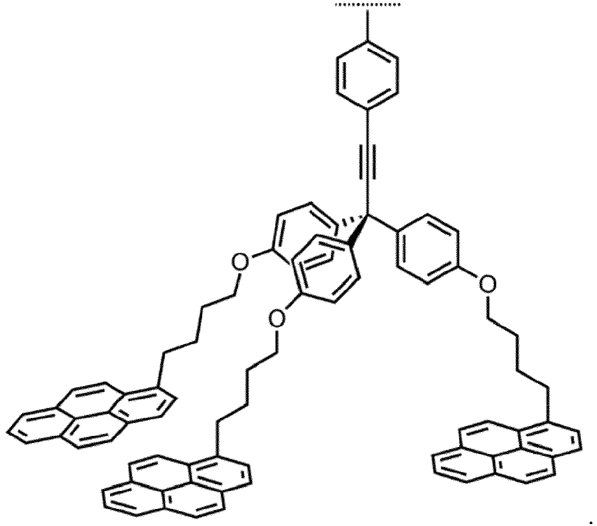

4. Ensemble selon l'une quelconque des revendications précédentes, dans lequel :
- Y est choisi parmi les anticorps anti Escherichia coli, les anticorps anti bactéries gram positives, les anticorps anti bactéries gram négatives et la concanavaline A, Y étant notamment un anticorps anti Escherichia coli ; et/ou
- Z est choisi parmi les cécropines, les défensines, les magainines et les dermaseptines, Z étant notamment une cécropine.

5. Ensemble selon l'une quelconque des revendications précédentes, dans lequel la densité en composés de formule (I) et (II) à la surface de l'ensemble d'atomes de carbone à l'état d'hybridation sp² est comprise d' 1 de ces composés par surface de 300 nm² à 1 de ces composés par surface de 2 nm², notamment 1 composé par surface de 2,7 nm² ± 10 %.

6. Ensemble selon l'une quelconque des revendications précédentes, dans lequel :
- le rapport molaire de la quantité de composés de formule (I) sur celle de composés de formule (II) est compris de 0,01 à 100, notamment de 0,1 à 10, en particulier de 0,33 à 1,5, plus particulièrement de 0,33 à 0,66 ou de 0,66 à 1,5, par exemple 0,5 ± 10 % ou 1,0 ± 10 %;
- l'ensemble d'atomes de carbone à l'état d'hybridation sp² est du graphène sous la forme de feuille, le rapport molaire de la quantité de composés de formule (I) sur celle de composés de formule (II) étant compris de 0,66 à 1,5, par exemple 1,0 ± 10 %; et/ou
- l'ensemble d'atomes de carbone à l'état d'hybridation sp² est du graphène sous la forme de flocons, le rapport molaire de la quantité de composés de formule (I) sur celle de composés de formule (II) étant compris de 0,33 à 0,66, par exemple 0,5 ± 10 %.

7. Procédé de préparation d'un ensemble d'atomes de carbone à l'état d'hybridation sp² selon l'une quelconque des revendications 1 à 6, comprenant les étapes suivantes :
(i) Mise en contact d'un ensemble d'atomes de carbone à l'état d'hybridation sp² avec un composé de formule (III) suivante :
(R-L₁-W)ₙ-V-L₂-X-Q (III),
dans laquelle :
R, L₁, W, V et L₂ sont tels que définis dans la revendication 1;
X-Q est un groupe choisi parmi -C(=O)O-*N*-succinimidyle, -C(=O)-halogènure, en particulier -C(=O)-Cl, -C(=O)-N₃, -C(=O)-O-*N*-imidazolyle, -C(=O)-O-C(=O)-R', avec R' étant un C₁-C₁₂ alkyle linéaire, ramifié ou cyclique, -C(=O)-O-catécholborane, -C(=O)-O-benzotriazole, en particulier -C(=O)O-*N*-succinimidyle,
pour obtenir un ensemble d'atomes de carbone à l'état d'hybridation sp² activé ;
(ii) Mise en contact de l'ensemble d'atomes de carbone à l'état d'hybridation sp² activé tel qu'obtenu à l'étape précédente avec une composition comprenant un anticorps antibactérien ou une lectine et un peptide antibactérien, pour obtenir un ensemble d'atomes de carbone à l'état d'hybridation sp² fonctionnalisé.

8. Utilisation d'un ensemble d'atomes de carbone à l'état d'hybridation sp² selon l'une quelconque des revendications 1 à 6, pour rendre une surface antibactérienne, en particulier bactéricide ou bactériostatique.

9. Matériau comprenant un substrat dont tout ou partie de la surface est recouverte d'un ensemble d'atomes de carbone à l'état d'hybridation sp² au contact de composés de formule (I) et (II) selon l'une quelconque des revendications 1 à 6.

10. Matériau selon la revendication 9, dans lequel ledit ensemble d'atomes de carbone à l'état d'hybridation sp2 est supporté par un substrat, notamment choisi parmi les métaux, les oxydes métalliques, les verres et les polymères.

11. Procédé de préparation d'un matériau selon l'une quelconque des revendications 9 à 10, comprenant les étapes suivantes :
(i) Mise en contact d'un ensemble d'atomes de carbone à l'état d'hybridation sp² avec un composé de formule (III) suivante :
(R-L₁-W)ₙ-V-L₂-X-Q (III),
dans laquelle :
R, L₁, W, V et L₂ sont tels que définis dans la revendication 1;
X-Q est un groupe choisi parmi -C(=O)O-*N*-succinimidyle, -C(=O)-halogènure, en particulier -C(=O)-Cl, -C(=O)-N₃, -C(=O)-O-*N*-imidazolyle, -C(=O)-O-C(=O)-R', avec R' étant un C₁-C₁₂ alkyle linéaire, ramifié ou cyclique, -C(=O)-O-catécholborane, -C(=O)-O-benzotriazole, en particulier -C(=O)O-*N*-succinimidyle,
pour obtenir ensemble d'atomes de carbone à l'état d'hybridation sp² activé;
(ii) Mise en contact de l'ensemble d'atomes de carbone à l'état d'hybridation sp² activé tel qu'obtenu à l'étape précédente avec une composition comprenant un anticorps antibactérien ou une lectine et un peptide antibactérien, pour obtenir un ensemble d'atomes de carbone à l'état d'hybridation sp² fonctionnalisé ;
(iii) Dépôt de l'en ensemble d'atomes de carbone à l'état d'hybridation sp² fonctionnalisé tel qu'obtenu à l'étape précédente sur la surface d'un support pour obtenir ledit matériau.

12. Procédé de préparation d'un matériau selon l'une quelconque des revendications 9 à 10, comprenant les étapes suivantes :
(i) Mise en contact d'un ensemble d'atomes de carbone à l'état d'hybridation sp² porté par la surface d'un matériau avec un composé de formule (III) suivante :
(R-L₁-W)ₙ-V-L₂-X-Q (III),
dans laquelle :
R, L₁, W, V et L₂ sont tels que définis dans la revendication 1;
X-Q est un groupe choisi parmi -O-*N*-succinimidyle, -halogènure, en particulier -Cl, -N₃, -O-*N*-imidazolyle, -O-C(=O)-R', avec R' étant un C₁-C₁₂ alkyle linéaire, ramifié ou cyclique, -O-catécholborane, -O-benzotriazole,
pour obtenir un matériau activé ;
(ii) Mise en contact du matériau activé tel qu'obtenu à l'étape précédente avec une composition comprenant un anticorps antibactérien ou une lectine et un peptide antibactérien, pour obtenir ledit matériau, fonctionnalisé.

## Patentansprüche

1. Funktionalisierte Kohlenstoffanordnung in sp²-Hybridisierungszustand, wobei die Kohlenstoffanordnung im sp²-Hybridisierungszustand in Kontakt steht mit:
- einer Vielzahl von Verbindungen der folgenden Formel (I):
(R-L₁-W)ₙ-V-L₂-X-Y (I),
worin:
n gleich 1, 2 oder 3 ist, insbesondere 3;
V für jedes n -C(H)₃₋ₙ- darstellt oder:
- wenn n gleich 2 ist, -C(H)=, wobei C in diesem Fall durch eine Doppelbindung an L₂ gebunden ist;
- wenn n gleich 1 ist, -C=, wobei C in diesem Fall durch eine Dreifachbindung an L₂ gebunden ist;
R ein aromatischer Kohlenwasserstoff ist, der 2 bis 6 kondensierte aromatische Ringe umfasst;
L₁ eine C₁-C₁₂-Alkandiylgruppe;
W eine Einfachbindung, eine Arendiylgruppe, eine Heteroarendiylgruppe oder eine Gruppe -O-Ar₁- darstellt, wobei Ar₁ ein Aren oder ein Heteroaren ist;
L₂ eine Gruppe der folgenden Formel -(L₂ₐ)ᵢ-(L_{2b})ⱼ- worin:
i und j unabhängig voneinander aus 0 und 1 ausgewählt sind, wobei i + j = 1 oder 2;
L₂ₐ eine Gruppe C₁-C₁₂ Alkandiyl, C₂-C₁₂ Alkendiyl oder C₂-C₁₂ Alkindiyl ist;
L_{2b} eine Arendiylgruppe, eine Heteroarendiylgruppe oder eine Gruppe -O-Ar₂- ist, wobei Ar₂ ein Aren oder ein Heteroaren ist;
X eine Gruppe -C(=O)N-, -C(=O)O- oder -C(=O)S- ist;
Y ein antibakterieller Antikörper oder ein Lektin ist;
und
- eine Vielzahl von Verbindungen der folgenden Formel (II):
(R-L₁-W)ₙ-V-L₂-X-Z (II),
worin:
R, L₁, W, V, L₂ und X wie oben definiert sind;
Z ein antibakterielles Peptid ist.

2. Anordnung nach Anspruch 1, wobei die Kohlenstoffatomanordnung im sp²-Hybridisierungszustand ausgewählt ist aus Graphen, Graphenoxid, Graphit, Kohlenstoffnanoröhren, Fullerenen und Fulleriten, wobei die Anordnung insbesondere Graphen ist, speziell in Form von Blättern oder Flocken

3. Anordnung nach einem der vorstehenden Ansprüche, wobei:
- R 1-Pyrenyl oder 2-Pyrenyl ist, insbesondere 1-Pyrenyl;
- L₁ eine Gruppe C₂-C₈-Alkandiyl; und/oder
- W eine Gruppe -O-Ar₁- darstellt, wobei Ar₁ ein Aren oder ein Heteroaren, insbesondere Benzol, ist; und/oder
- i und j gleich 1 sind; und/oder
- L₂ₐ eine Gruppe C₂-C₁₂-Alkindiyl, insbesondere -C=C- ist; und/oder
- L_{2b} eine Arendiylgruppe, insbesondere Benzoldiyl, ist; und/oder
- R, L₁, W, V und L₂ wie in der folgenden Formel definiert sind:

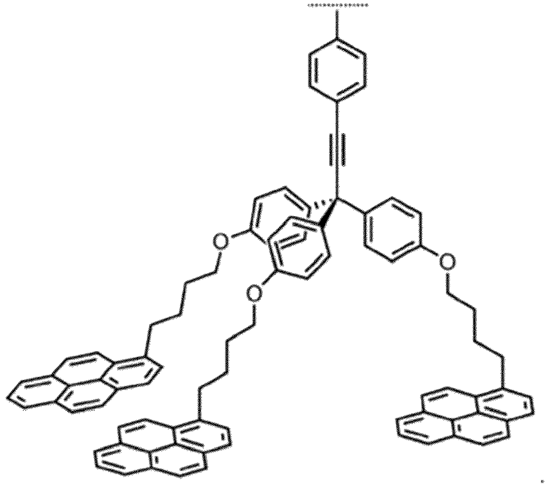

4. Anordnung nach einem der vorstehenden Ansprüche, wobei:
- Y ausgewählt ist aus die Antikörpern gegen Escherichia coli, die Antikörpern gegen grampositive Bakterien, die Antikörpern gegen gramnegative Bakterien und die Concanavalin A, wobei Y insbesondere ein Antikörper gegen Escherichia coli ist; und/oder
- Z ausgewählt ist aus die Cecropinen, die Defensinen, die Magaininen und die Dermaseptinen, wobei Z insbesondere ein Cecropin ist.

5. Anordnung nach einem der vorstehenden Ansprüche, wobei die Dichte an Verbindungen der Formeln (I) und (II) auf der Oberfläche der Kohlenstoffatomanordnung im sp²-Hybridisierungszustand von 1 dieser Verbindungen pro 300 nm² Oberfläche bis 1 dieser Verbindungen pro 2 nm² Oberfläche, insbesondere 1 Verbindung pro 2,7 nm² Oberfläche ± 10 % beträgt.

6. Anordnung nach einem der vorstehenden Ansprüche, wobei:
- das Molverhältnis der Menge der Verbindungen der Formel (I) zu derjenigen der Verbindungen der Formel (II) 0,01 bis 100, besonders 0,1 bis 10, insbesondere 0,33 bis 1,5, speziell 0,33 bis 0,66 oder 0,66 bis 1,5, zum Beispiel 0,5 ± 10 % oder 1,0 ± 10 %; beträgt;
- die Kohlenstoffatomanordnung im sp²-Hybridisierungszustand aus Graphen in Blattform besteht, wobei das Molverhältnis der Menge der Verbindungen der Formel (I) zu derjenigen der Verbindungen der Formel (II) von 0,66 bis 1,5, zum Beispiel 1,0 ± 10 %, beträgt, und/oder
- die Kohlenstoffatomanordnung im sp²-Hybridisierungszustand aus Graphen in Flockenform besteht, wobei das Molverhältnis der Menge der Verbindungen der Formel (I) zu derjenigen der Verbindungen der Formel (II) 0,33 bis 0,66, zum Beispiel 0,5 ± 10 %, beträgt.

7. Verfahren zur Herstellung einer Kohlenstoffatomanordnung im sp²-Hybridisierungszustand nach einem der Ansprüche 1 bis 6, umfassend die folgenden Schritte:
(i) In Kontakt bringen einer Kohlenstoffatomanordnung im sp²-Hybridisierungszustand mit einer Verbindung der folgenden Formel (III):
(R-L₁-W)ₙ-V-L₂-X-Q (III),
worin:
R, L₁, W, V und L₂ wie in Anspruch 1 definiert sind;
X-Q eine Gruppe ist, ausgewählt aus -C(=O)O-*N*-Succinimidyl, -C(=O)-Halogenid, insbesondere-C(=O)-CI, -C(=O)-N₃, -C(=O)-O-*N*-Imidazolyl, -C(=O)-O-C(=O)-R', wobei R' ein lineares, verzweigtes oder cyclisches C₁-C₁₂-Alkyl, -C(=O)-O-Catecholboran, - C(=O)-O-Benzotriazol, insbesondere -C(=O)O-*N*-Succinimidyl ist,
um eine Kohlenstoffatomanordnung im aktivierten sp²-Hybridisierungszustand zu erhalten;
(ii) In Kontakt bringen der Kohlenstoffatomanordnung in aktiviertem sp²-Hybridisierungszustand, wie im vorstehenden Schritt erhalten, mit einer Zusammensetzung, die einen antibakteriellen Antikörper oder ein Lektin und ein antibakterielles Peptid umfasst, um eine funktionalisierte Kohlenstoffatomanordnung in sp²-Hybridisierungszustand zu erhalten.

8. Verwendung einer Kohlenstoffatomanordnung im sp²-Hybridisierungszustand nach einem der Ansprüche 1 bis 6, um eine antibakterielle, insbesondere bakterizide oder bakteriostatische Oberfläche zu erzeugen.

9. Material, umfassend ein Substrat, dessen Oberfläche ganz oder teilweise mit einer Kohlenstoffatomanordnung im sp²-Hybridisierungszustand bei Kontakt mit Verbindungen der Formeln (I) und (II) nach einem der Ansprüche 1 bis 6 bedeckt ist.

10. Material nach Anspruch 9, wobei die Kohlenstoffatomanordnung im sp²-Hybridisierungszustand von einem Träger getragen wird, der insbesondere aus Metallen, Metalloxiden, Gläsern und Polymeren ausgewählt ist.

11. Verfahren zur Herstellung eines Materials nach einem der Ansprüche 9 bis 10, umfassend die folgenden Schritte:
(i) In Kontakt bringen einer Kohlenstoffatomanordnung im sp²-Hybridisierungszustand mit einer Verbindung der folgenden Formel (III):
(R-L₁-W)ₙ-V-L₂-X-Q (III),
worin:
R, L₁, W, V und L₂ wie in Anspruch 1 definiert sind;
X-Q eine Gruppe ist, ausgewählt aus -C(=O)O-*N*-Succinimidyl, -C(=O)-Halogenid, insbesondere -C(=O)-CI, -C(=O)-N₃, -C(=O)-O-*N*-Imidazolyl, -C(=O)-O-C(=O)-R', wobei R' ein lineares, verzweigtes oder cyclisches C₁-C₁₂-Alkyl, -C(=O)-O-Catecholboran, - C(=O)-O-Benzotriazol, insbesondere -C(=O)O-*N*-Succinimidyl ist,
um eine Kohlenstoffatomanordnung im aktivierten sp²-Hybridisierungszustand zu erhalten;
(ii) In Kontakt bringen der Kohlenstoffatomanordnung in aktiviertem sp²-Hybridisierungszustand, wie im vorstehenden Schritt erhalten, mit einer Zusammensetzung, die einen antibakteriellen Antikörper oder ein Lektin und ein antibakterielles Peptid umfasst, um eine funktionalisierte Kohlenstoffatomanordnung in sp²-Hybridisierungszustand zu erhalten;
(iii) Ablagerung der funktionalisierten Kohlenstoffatomanordnung in sp²-Hybridisierungszustand, wie sie im vorstehenden Schritt erhalten wurde, auf der Oberfläche eines Trägers, um das Material zu erhalten.

12. Verfahren zur Herstellung eines Materials nach einem der Ansprüche 9 bis 10, umfassend die folgenden Schritte:
(i) In Kontakt bringen einer Kohlenstoffatomanordnung im sp²-Hybridisierungszustand, die von der Oberfläche eines Materials getragen wird, mit einer Verbindung der folgenden Formel (III) :
(R-L₁-W)ₙ-V-L₂-X-Q (III),
worin:
R, L₁, W, V und L₂ wie in Anspruch 1 definiert sind;
X-Q eine Gruppe ist, ausgewählt aus -C(=O)O-*N*-Succinimidyl, -Halogenid, insbesondere -C(=O)-CI, -C(=O)-N₃, -C(=O)-O-*N*-Imidazolyl, -C(=O)-O-C(=O)-R', wobei R' ein lineares, verzweigtes oder cyclisches C₁-C₁₂-Alkyl, -O-Catecholboran, -O-Benzotriazol ist, um ein aktiviertes Material zu erhalten;
(ii) In Kontakt bringen des aktivierten Materials, wie im vorstehenden Schritt erhalten, mit einer Zusammensetzung, die einen antibakteriellen Antikörper oder ein Lektin und ein antibakterielles Peptid umfasst, um das funktionalisierte Material zu erhalten.

## Claims

1. A functionalized set of carbon atoms in the sp² hybridization state, said set of carbon atoms in the sp² hybridization state being in contact with:
- a plurality of compounds of the following formula (I):
(R-L₁-W)ₙ-V-L₂-X-Y (I),
wherein:
n is equal to 1, 2, or 3, in particular 3;
V represents, for all n, -C(H)₃₋ₙ- or:
- when n is equal to 2, -C(H)=, C being in this case linked to L₂ by a double bond;
- when n is equal to 1, -C=, C being in this case linked to L₂ by a triple bond;
R is an aromatic hydrocarbon comprising from 2 to 6 condensed aromatic rings;
L₁ is a C₁-C₁₂ alkanediyl group;
W represents a single bond, an arenediyl group, a heteroarenediyl group or a -O-Ar₁- group wherein Ar₁ is an arene or a heteroarene;
L₂ is a group of the following formula (A) -(L₂ₐ)ᵢ-(L_{2b})ⱼ₋ wherein:
i and j are independently of each other selected from 0 and 1, with i + j = 1 or 2;
L₂ₐ is a C₁-C₁₂ alkanediyl, C₂-C₁₂ alkenediyl or C₂-C₁₂ alkynediyl group;
L_{2b} is an arenediyl group, a heteroarenediyl group or a -O- Ar₂- group where Ar₂ is an arene or a heteroarene;
X is -C(=O)N-, -C(=O)O- or -C(=O)S- group;
Y is an antibacterial antibody or a lectin;
and
- a plurality of compounds of the following formula (II):
(R-L₁-W)ₙ-V-L₂-X-Z (II),
wherein:
R, L₁, W, V, L₂ and X are as defined above;
Z is an antibacterial peptide.

2. The set according to claim 1, wherein said set of carbon atoms in the sp² hybridization state is selected from graphene, graphene oxide, graphite, carbon nanotubes, fullerenes and fullerites, said set being in particular graphene, more particularly in the form of a sheet or flakes.

3. The set according to any one of the preceding claims, wherein:
- R is the 1-pyrenyl or the 2-pyrenyl, in particular the 1-pyrenyl;
- L₁ is a C₂-C₈ alkanediyl group; and/or
- W represents a group -O-Ar₁- where Ar₁ is an arene or a heteroarene, in particular a benzene; and/or
- i and j are equal to 1; and/or
- L₂ₐ is a C₂-C₁₂ alkynediyl group, in particular -C=C-; and/or
- L_{2b} is an arenediyl group, in particular a benzenediyl; and/or
- R, L₁, W, V and L₂ are as defined by the following formula:

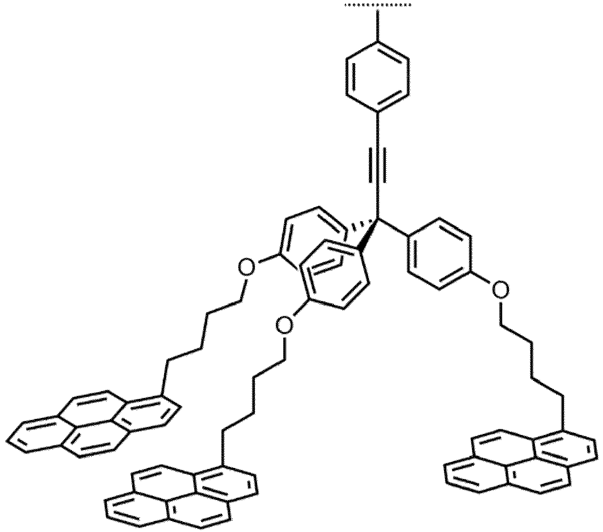

4. The set according to any one of the preceding claims, wherein:
- Y is selected from the anti-Escherichia coli antibodies, the anti-gram-positive bacteria antibodies, the anti-gram-negative bacteria antibodies and the concanavalin A, Y being in particular an anti-Escherichia coli antibody; and/or
- Z is selected from cecropins, defensins, magainins and dermaseptins, Z being in particular a cecropin.

5. The set according to any one of the preceding claims, wherein the density of compounds of formulae (I) and (II) on the surface of the set of carbon atoms in the sp² hybridization state ranges from 1 of these compounds per 300 nm² area to 1 of these compounds per 2 nm² area, in particular 1 compound per 2.7 nm² area ± 10%.

6. The set according to any one of the preceding claims, wherein:
- the molar ratio of the amount of compounds of formula (I) to that of compounds of formula (II) is comprised from 0.01 to 100, in particular from 0.1 to 10, in particular from 0.33 to 1.5, more particularly from 0.33 to 0.66 or from 0.66 to 1.5, for example 0.5 ± 10% or 1.0 ±10%;
- the set of carbon atoms in the sp² hybridization state is graphene in the form of sheet, the molar ratio of the amount of compounds of formula (I) to that of compounds of formula (II) being comprised from 0.66 to 1.5, for example 1.0 ± 10%; and/or
- the set of carbon atoms in the sp² hybridization state is graphene in the form of flakes, the molar ratio of the amount of compounds of formula (I) to that of compounds of formula (II) being comprised from 0.33 to 0.66, for example 0.5 ± 10%.

7. A method of preparing a set of carbon atoms in the sp² hybridization state according to any one of claims 1 to 6, comprising the following steps:
(i) Contacting a set of carbon atoms in the sp² hybridization state with a compound of the following formula (III) :
(R-L₁-W)ₙ-V-L₂-X-Q (III),
wherein:
R, L₁, W, V and L₂ are as defined in claim 1;
X-Q is a group selected from -C(=O)O-*N*-succinimidyl, -C(=O)-halide, in particular -C(=O)-CI, -C(=O)-N₃, -C(=O)-O-*N*-imidazolyl, -C(=O)-O-C(=O)-R', with R' being a linear, branched or cyclic C₁-C₁₂ alkyl, -C(=O)-O-catecholborane, -C(=O)-O-benzotriazole, in particular -C(=O)O-*N*-succinimidyl,
to obtain a set of carbon atoms in the activated sp² hybridization state;
(ii) Contacting the set of carbon atoms in the activated sp² hybridization state as obtained in the previous step with a composition comprising an antibacterial antibody or a lectin and an antibacterial peptide, to obtain a functionalised set of carbon atoms in the sp² hybridization state.

8. Use of a set of carbon atoms in the sp² hybridization state according to any one of claims 1 to 6, for rendering a surface antibacterial, in particular bactericidal or bacteriostatic.

9. A material comprising a substrate all or part of the surface of which is covered with a set of carbon atoms in the sp² hybridization state in contact with compounds of formulae (I) and (II) according to any of claims 1 to 6.

10. The material according to claim 9, wherein said set of carbon atoms in the sp² hybridization state is supported by a substrate, in particular selected from metals, metal oxides, glasses and polymers.

11. A method for preparing a material according to any one of claims 9 to 10, comprising the following steps:
(i) Contacting a set of carbon atoms in the sp² hybridization state with a compound of the following formula (III):
(R-L₁-W)ₙ-V-L₂-X-Q (III),
wherein:
R, L₁, W, V and L₂ are as defined in claim 1;
X-Q is a group selected from -C(=O)O-*N*-succinimidyl, -C(=O)-halide, in particular -C(=O)-CI, -C(=O)-N₃, -C(=O)-O-*N*-imidazolyl, -C(=O)-O-C(=O)-R', with R' being a linear, branched or cyclic C₁-C₁₂ alkyl, -C(=O)-O-catecholborane, -C(=O)-O-benzotriazole, in particular - C(=O)O-*N*-succinimidyl,
to obtain a set of carbon atoms in the activated sp² hybridization state;
(ii) Contacting the set of carbon atoms in the activated sp² hybridization state as obtained in the previous step with a composition comprising an antibacterial antibody or a lectin and an antibacterial peptide, to obtain a functionalized set of carbon atoms in the sp² hybridization state;
(iii) Depositing the functionalized set of carbon atoms in the sp² hybridization state as obtained in the previous step on the surface of a support to obtain said material.

12. A method for preparing a material according to any one of claims 9 to 10, comprising the following steps:
(i) Contacting a set of carbon atoms in the sp² hybridization state carried by the surface of a material with a compound of the following formula (III):
(R-L₁-W)ₙ-V-L₂-X-Q (III),
wherein:
R, L₁, W, V and L₂ are as defined in claim 1;
X-Q is a group selected from -O-*N*-succinimidyl, -halide, in particular -Cl, -N₃, -O-*N-*imidazolyl, -O-C(=O)-R', with R' being a linear, branched or cyclic C₁-C₁₂ alkyl, -O-catecholborane, -O-benzotriazole,
to obtain an activated material;
(ii) Contacting the activated material as obtained in the previous step with a composition comprising an antibacterial antibody or a lectin and an antibacterial peptide, to obtain said functionalized material.
